(19) Europäisches Patentamt
European Patent Office
Office européen des brevets

(11) EP 3 505 166 A1

(12) EUROPEAN PATENT APPLICATION
published in accordance with Art. 153(4) EPC

(43) Date of publication:
03.07.2019 Bulletin 2019/27

(21) Application number: 16914318.7

(22) Date of filing: 24.08.2016

(51) Int Cl.:
*A61K 9/70* (2006.01) *A61K 33/08* (2006.01)
*A61K 33/24* (2019.01) *A61P 31/04* (2006.01)
*B82Y 5/00* (2011.01)

(86) International application number:
PCT/RU2016/000567

(87) International publication number:
WO 2018/038627 (01.03.2018 Gazette 2018/09)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicants:
- Kamler, Anna Vladimirovna
Moscow 119330 (RU)
- Barinov, Andrei Konstantinovich
Moscow 119607 (RU)

(72) Inventors:
- Kamler, Anna Vladimirovna
Moscow 119330 (RU)
- Barinov, Andrei Konstantinovich
Moscow 119607 (RU)

(74) Representative: Lapienis, Juozas
MSP Europe UAB
21-92 Seimyniskiu Str.
09236 Vilnius (LT)

# (54) THREE-DIMENSIONAL ANTIBACTERIAL MATERIAL, METHOD FOR PREPARATION THEREOF (VARIANTS), AND UNIT FOR IMPLEMENTING THE METHOD

(57) The invention relates to a new antibacterial material and a method of preparing same, which is presented in variants, as well as for a unit for implementing the above-named method. A three-dimensional antibacterial material is a microfiber matrix with chemically or physically fastened nanodimensional compositions on the fibers thereof, said nanodimensional compositions being nanoparticles of titanium oxide (TiO2) or compositions containing nanoparticles of titanium oxide and nanoparticles of oxides of other metals or compounds thereof. The invention is aimed at developing processes for applying durable nanocoatings having antibacterial properties to textiles, which can be used for prophylaxis of nosocomial infections.

EP 3 505 166 A1

## Description

The invention relates to a new antibacterial material, a method for production thereof provided in variants, as well as to a unit for implementing the above-mentioned method. The invention is aimed at developing processes for applying durable nanocoatings having antibacterial properties to textiles, which can be used for prophylaxis of nosocomial infections.

Nosocomial infections are infections acquired while receiving treatment or when working in a healthcare facility. According to an international classification nosocomial infections are divided into 13 classes and 50 types based on clinical and biological criteria. Microorganisms that are usually associated with nosocomial infections include streptococci, Acinetobacter SPP, Enterococci, Pseudomonas aeruginosa, coagulase staphylococci, Staphylococcus aureus, Bacillus Cereus, Legionella and Enterobacteriaceae namely, Proteus mirablis, Klebsiella pneumoniae, colon bacillus, Serratia marcescens. Infectious agents of the nosocomial infections can be spread from one person to another through the environment or through contaminated water and food, through skin contact or through contaminated objects of the healthcare personnel or via objects and surfaces of shared use, most often textiles. An excessive and inappropriate use of wide-spectrum antibiotics, especially in healthcare facilities, aggravates the problem of nosocomial infections, which along with harm to health cause serious economic damage. An inappropriate use of antibiotics often leads to the evolution of microorganisms, which become drug-resistant. Such organisms include nosocomial methicillin-resistant Staphylococcus aureus, vancomycin-resistant Enterococci, vancomycin-resistant Pseudomonas aeruginosa and vancomycin-resistant Klebsiella pneumoniae [Asian Pacific Journal of Tropical Biomedicine, Volume 5, Issue 7, July 2015, pp. 509-514].

The contemporary market of bactericide textile materials in Europe is limited owing to their high price. This is partly attributable to the fact that mainly silver is used as an antibacterial agent in production of currently commercially available antibacterial textile materials.

A coating in which a layer of particles from at least one nanoscale photocatalytic material is applied onto the primary porous layer from an inorganic or an inorganic and organic material is known, wherein particles of an average size from 2 nm to 150 nm are spaced from each other at a distance from 1 nm to 50 nm on the surface of the primary layer. The primary layer of the coating can be made of SiO2, ZrO, A12O3, as well as of mixed oxides of these elements or modified organic compounds of these elements. Such a coating can be applied onto synthetic materials, textile, ceramics, wood, metal or lacquer. (DE 101 58 433 B4 2006.05.18).

A method for preparing (DE 10 2006 053 326 A1 2008.05.15) a coating for hydrophobic or oleophobic surface finishing is also known, in which a dispersive material and active particles with hydrophobic or oleophobic surface groups dispersed therein and a binder are used. In this case for activation the particles with hydrophobic or oleophobic surface groups are put into the dispersive material to be ground, and binder is added before or after activation.

Such a coating leads to a noticeable improvement in physical properties of a transparent layer along with good hydrophobic or oleophobic properties. It is suitable for application on any surfaces, in particular, on fibers and any textile, on glass, ceramics, metal, wood and synthetic substances.

Various types of particle grinding (both chemical and physical) in a dispersive material are described according to the method. In this case particles themselves can be metal oxides and combinations thereof (including TiO2).

Herein a coating for imparting hydrophobic or oleophobic properties to the surfaces is claimed, which contains a dispersive material, hydrophobic or oleophobic particles dispersed therein and a binder.

The prior art discloses a device for impregnation of a moving fabric (the RF patent No. 2157868). The device comprises a bath for the impregnating liquid, two or more conveying drums, arranged in series along the path of the fabric being impregnated in the bath, two endless belts for conveying the fabric which are disposed on carrier rollers and are made in the form of a mesh, and plates immersed into the bath and installed both above and under the fabric movement plane. The plates are oriented at an angle to the fabric movement plane and are arranged to change said angle and the immersion depth, which enables to choose an optimum mode of moistening the fabric due to the adjustment of the wave formation processes of in the layers adjacent to the belts and in the water surface layer.

The closest to the claimed invention are the textile material and the method for forming thereof described in the PRC invention patent application No. 200610049069.6. According to the invention a method is suggested for production in situ of inorganic nanoscale particles of titanium (IV) oxide in a textile material: at a standard temperature while stirring titanium (IV) chloride is added to ethanol solution comprising triethanolamine to form a paste-like complex compound. Deionized water is added to the complex compound to be solved held at a temperature of approximately 30 °C, to obtain a transparent solution. The resulting solution while stirring or ultrasonic dispersing is mixed with 1-5 % solution of amino alcohol AMP-95 (2-amino-methyl-1-propanol) in water with the textile material to be fully impregnated with the solution, and pH adjustment is performed. When ultrasonic or hydrothermal method is used nanoscale particles of titanium (IV) oxide are formed in situ in the fibers of textile material fabric.

The substance of titanium (IV) chloride and triethanolamine is taken in proportion of 1: 2; ethanol is used as a solvent, its volume being three times larger than that of triethanolamine.

The Hybrid System is loaded into a hydrothermal autoclave for a treatment using hydrothermal method, at a

reaction temperature of 145-200°C nanoscale particles of titanium (IV) oxide in the form of crystalline anatase are created in situ in the fibers.

Due to a sufficiently strong positive electrical charge of titanium (IV) Ti + ion, a fast hydrolysis occurs at a standard temperature with formation of titanium hydroxide, from which titanium (IV) oxide is obtained after calcining. When a stable complex compound is formed by triethanolamine and titanium Ti + ions of the positive electrical charge Ti4+ is neutralized, which reduces the hydrolysis rate.

Hydrolysis can occur in the two ways:

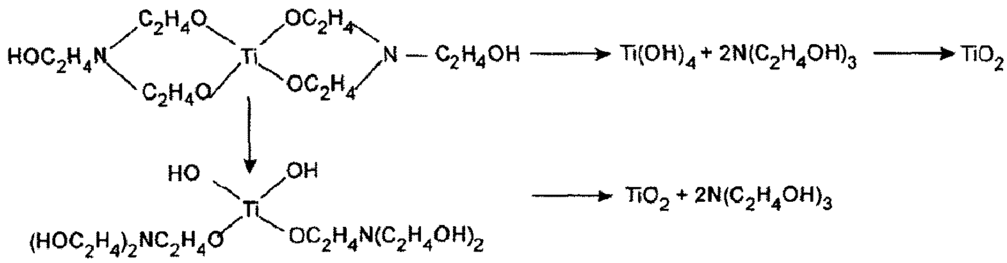

The first possible way of hydrolysis contemplates destruction of all the four Ti-O bonds during hydrolysis to form titanium hydroxide. Another possible way contemplates but partial destruction of Ti-O bonds during hydrolysis subsequently forming TiO2 directly when triethanolamine is separated from the molecule.

The method for forming TiO2 or SiO2 inorganic nanoscale particles in situ in a textile material enhances dispersity characteristics and adhesion of the nanoparticles in the fabric with the purpose of improving the characteristics of the textile articles in terms of protection against ultraviolet radiation and bacteria.

However, the method enables to obtain an antibacterial material only through covering of titanium dioxide or silica with nanoparticles. This limits possibilities of using different compositions of titanium oxide and other metal oxides (such as MgO, ZnO, CuO, CeO2) and, as a consequence, limits the development of market segments.

The object of the invention as claimed is preparation of a more efficient antibacterial material based on the available metal oxides and their compositions and development of a textile treatment technique.

The problem set is solved by means of a new three-dimensional antibacterial material designed for production of wound treatment bandages for textile articles with antibacterial properties, being a microfiber matrix with nanoscale compositions chemically or physically attached to its fibers with nanoscale compositions being represented by nanoparticles of titanium oxide (TiO2) or compositions containing nanoparticles of titanium oxide and nanoparticles of oxides of other metals or compounds thereof, wherein the content of nanoparticles in the material structure complies with the following inequality:

$$1{,}1 \leq \frac{\sum X_i}{\sum Y_i} \leq 100$$

, where

- Xi - a total concentration of nanoparticles of titanium oxide TiO2
- Yi - a total concentration of nanoparticles of oxides of other metals or compounds thereof,

the number of nanoscale particles being from 0.1 % to 30 % (wt). The nanoparticle size in the material is from 1 nm to 500 nm.

The three-dimensional antibacterial material contains ZnO, CuO, MgO, CeO2 as nanoparticles of oxides of other metals or compounds thereof.

The lower limit 1.1 in the inequality $1{,}1 \leq \frac{\sum X_i}{\sum Y_i} \leq 100$ provides a higher content of nanoparticles of titanium TiO 2 oxide than the total concentration of other metals or their compounds. The upper limit 100 in the inequality limits the minimum content of other metals compared to the nanoparticles of titanium oxide TiO2 in the range of 1 %.

To decrease the cost of the product under development in comparison with the prototype and to increase antibacterial activity of the material alternative materials having antibacterial properties were examined. Nonetheless,

during examination a special focus was made on the materials having a good biological compatibility with human skin.

Also the problem is solved by using the method for preparing a three-dimensional antibacterial textile material provided in variants.

Further, the problem is solved by means of a device for implementation of the above-mentioned method.

In the first embodiment of the method for preparing three-dimensional antibacterial material a titanium-containing solution or a solution, containing compounds of other metals (Zn, Cu, Mg, Ce etc.) along with said titanium-containing compounds, which serve as starting compounds for forming nanoparticles of such metal oxides, are prepared beforehand. Afterwards the textile material is impregnated with the solution prepared under ultrasonic vibrations at a frequency from 10 kHz to 100 kHz until the fibers are fully moistened and impregnated with the solution prepared. Subsequent formation of sol from the initial solution is performed. It can be performed by heating, preliminary drying, water vapour treatment, adding a substance for pH adjusting/modifying or other reagents. A gel is formed. After washing the material with water a temperature control is performed at a temperature from 80 to 200 Celsius degrees.

The second method for production of a three-dimensional antibacterial textile material, includes preliminary impregnation of the textile material with a reagent having a pH different from a neutral one, preparation of a titanium-containing solution (the solution may contain compounds of other metals (Zn, Cu, Mg, Ce etc.) along with titanium-containing compounds , which serve as starting compounds for forming nanoparticles of such metal oxides); impregnation of the textile material with the solution prepared under ultrasonic vibrations at a frequency from 10 kHz to 100 kHz which provide full moistening and impregnation of the fibers with the solution prepared, wherein due to a different pH (obtained through preliminary impregnation) a nanosol and a gel are formed on the fibers, washing the material with water and a final drying of the material at a temperature from 80 to 200 Celsius degrees.

According to the claimed invention nanoparticles are formed directly in the fibers of textile materials. The main component of a three-dimensional antibacterial structure is a metal oxide. In a textile material nanoscale particles are chemically bound with the textile fibers. Cotton or a cotton-containing material are used as a textile, a chemical bond is formed between the molecules of cellulose included in cotton and nanoparticles of titanium oxide or oxides of other metals. The sizes of the nanoparticles are from 1 nm to 1000 nm. The spatial arrangement of nanoparticles relative to one another is determined by the geometry of the textile fibers since a bond with the molecules of cellulose is formed. Thus, uniformity of the arrangement of nanoparticles across the whole material is achieved. Also uniformity of the arrangement of nanoparticles on the fibers is achieved via ultrasonic exposure at a frequency of 10-100 kHz at a stage of the fabric fibers impregnation with a liquid titanium-containing solution. It is ultrasonic exposure that facilitates penetration of substances into the fibers and allows for forming a three-dimensional structure instead of a surface coating.

Thus, formation of a three-dimensional structure of a textile material containing nanoparticles on the surface of the fibers and inside thereof is ensured.

The first stage of the method for preparing a three-dimensional antibacterial textile material includes preparation of an initial solution and impregnation of the textile material with this solution under ultrasonic vibrations. A precavitation mode can be used which is an obvious advantage of the method compared to the technique described in [Materials Letters 96 (2013) 121-124], since it will enable to decrease the energy consumption of the unit and the influence on the textile (i.e. will enable to preserve durability of the material). As a result of chemical bonding of the composition obtained with the molecules of cellulose chemical and mechanical durability of the coating is achieved. Afterwards the textile material undergoes a secondary treatment, in particular, thermal treatment, in particular, by vapour, during which hydrolysis occurs and the sol is formed. The process can be controlled through the vapour temperature adjustment. Afterwards the sol is transformed into the gel when a part of water in it is removed by heating or extraction using a corresponding solvent. Heated air can be used to accomplish this. Solid gel particles form a loose spatial grid, which comprises in its cells a liquid dispersive material preventing the whole System from the flow behaviour.

After impregnation and secondary treatment, washing of the textile with water with a subsequent drying at a temperature up to 200 degrees Celsius are performed. The final hardening of the gel occurs during the final drying, and the process of forming a porous structure with antibacterial properties chemically linked with the fiber is completed.

The sol may be formed without using vapour, just through heating up to a threshold temperature. Heating can also be performed by means of ultrasonic vibrations, in this case before reaching the threshold temperature ultrasonic vibrations are used for material impregnation. When the threshold temperature is reached the sol, and then the gel begins to form around the fibers. In this case ultrasonic vibrations in the area of formation of the sol and the gel prevent nanoparticle agglomeration which facilitates a more uniform distribution of nanoparticles. The three-dimensional structure is finally fixed at the stage of drying.

Vapour is not used in the second embodiment of the method as well. One more stage of preliminary impregnation is added. The sol begins to form based on formation of certain pH around the fibers through preliminary impregnation of the textile with a reagent having pH different from the neutral one.

The second method for producing a three-dimensional antibacterial textile material includes impregnation of the textile material with a titanium-containing reagent, in which the textile material is preimpregnated with a reagent having a pH different from the neutral one, afterwards the textile material is treated with the titanium-containing reagent

under ultrasonic vibrations at a frequency from 10 kHz to 100 kHz until the fibers are completely moistened and impregnated, the nanosol and gel are formed on the fibers, the material is washed, mainly with water, and dried at a temperature from 80 200 degrees Celsius.

The method as claimed has the following advantages compared to the prototype:

- The method allows for using any metals and their compositions; compositions based on titanium dioxide, oxides of copper, zinc and manganese. These compounds are an alternative to silver which significantly lowers the cost of the technique. Oxides of metals which have a lower cost compared to conventional silver are used as an antibacterial agent.
- Due to the ultrasonic impregnation uniformity of nanoparticle distribution in the material is provided.
- A developed surface of the obtained material porous structure is achieved, which enables to increase antibacterial activity of the claimed material compared to the material obtained using the prototype method.
- The method enables to provide for durability of the coating through chemical linking of the nanoparticles. An abrasion-resistant coating is obtained.

The resultant antibacterial coating is durable and withstands at least 100 washings; there is no detachment of the coating on the skin or in water during the washing operation.

Antibacterial activity of the material containing titanium dioxide or its compositions after washing can be increased by sunlight treatment before use.

- A high speed of the process is ensured thanks to a high rate of the reaction.
- The precavitation mode allows for increasing the energy consumption and the influence on the material durability.

The new textile material durability is not lower than that of the material without antibacterial properties.

It should be noted that the antibacterial properties of the material are no lower than A=4 for the most widespread bacteria (in this case antibacterial activity is expressed by the factor A, which is introduced in the following way: A=1 when the number of bacteria drops by 90 %; A=2 when the number of bacteria drops by 99 %; A=3 when the number of bacteria drops by 99.9 % etc.).

A device for production of a three-dimensional antibacterial material comprises a reagent preparation unit, an impregnation unit,
wherein the reagent preparation unit comprises a container equipped with taps for adding liquid components, as well as dispensers for adding powder components, a mixing unit, as well as temperature measuring sensors, a temperature control unit for controlling and maintaining temperature below the temperature at which nanoparticles start forming,
wherein the impregnation unit comprises an impregnation container, a reagent heating unit, a temperature control sensor, ultrasonic transducers of a cylindrical or a flat shape, ensuring a uniform exposure of the transported textile on both sides to mechanical vibrations at a frequency 10-100 kHz provided by ultrasonic generators, a tape-transport mechanism for transporting the textile material, the impregnation unit comprising an even number of ultrasonic transducers shifted relative to one another such that the transverse exposure maximum of the nth transducer coincides with the transverse exposure minimum of the (n+1)th transducer, where n = 1,2,3. The device further comprises a washing unit and a drying unit, the textile washing unit comprises a mechanism providing barbotage of the liquid.

In the claimed device the reagent preparation unit is equipped with a container for storage of initial liquid reagents.

Besides, the device further comprises a secondary treatment unit comprising a vapour treatment module or a liquid reagent impregnation module or a special medium washing module comprising a container and a tape-transport mechanism or a module of preliminary drying at a lower temperature.

Also the device further comprises a preimpregnation unit placed upstream the impregnation unit, the preliminary impregnation unit comprising a container for impregnation and a tape-transport mechanism for transporting the textile material.

The device is an automatized production line for producing a metal oxide based nanoparticle composition in a textile material containing 30 %-100 % of cotton, comprising a System of units, including an initial solution preparation unit (containing compounds of other metals (Zn, Cu, etc.) along with titanium-containing compounds, which serve as starting compounds for forming nanoparticles of such metal oxides), a textile material impregnation unit, comprising a structure for transporting the textile material with a width of 50-500 cm, ultrasonic transducers of a cylindrical or a flat shape, a textile material secondary treatment unit, comprising a container for secondary treatment (heating, vapour treatment, or adding an additional reagent that facilitates separation of nanoparticles), a washing unit for washing the

textile material with water and a drying unit for drying at a temperature of 80-200 Celsius degrees, ultrasonic transducers configured for uniformity of the textile material impregnation with the initial solution which are arranged for the textile passing with its every section through the area of mechanical vibrations at a frequency of 10-100 kHz and are provided by ultrasonic generators.

In the the method according to the second embodiment instead of a secondary treatment container the device comprises a container for preliminary impregnation of the textile with a reagent having a different pH.

The device operates as follows. Fig. 1 illustrates a chart of transporting the material through the following units:

1- reagent preparation unit

2- temperature control unit

3- impregnation unit

4- secondary treatment unit

5- washing and drying unit

6- preliminary impregnation unit
Units 4 and 6 may be used additionally.

At the first stage reagent is prepared at the reagent preparation unit 1. With this purpose a reagent mixing container made of stainless steel and having a volume of 20-40 litres can be used. This container may comprise taps to add liquid reagents, as well as a dispenser to add powder reagents. Besides, the reagent preparation unit 1 comprises a mixing device and may comprise sensors for measuring temperature, pH and other parameters. Unit 2 comprises a temperature control device to control and maintain temperature below the temperature at which nanoparticles start forming. In addition, the unit may comprise containers for storage of the initial liquid reagents (before mixing). The reagent mixing container is closed and prevents formation of vapour and its discharge to the environment.

The impregnation unit 3 in addition to the impregnation container may comprise a temperature control device to maintain the required temperature of the process. The reagent prepared at the reagent preparation unit 1 may pass through a heating device in order to bring the temperature of the reagent closer to the threshold temperature for formation of metal oxide nanoparticles. In this case the temperature before impregnation remains below the threshold temperature. Ultrasonic transducers may be used for final increase of the reagent temperature after impregnation of the fabric.

The impregnation unit 3 comprises ultrasonic transducers provided by ultrasonic generators (more than one generator or a multichannel generator). The textile (possibly after preimpregnation) is supplied to the container for impregnation and passes between ultrasonic transducers in such a way as to provide for a uniform irradiation of the textile on both sides. For this reason, an even number of ultrasonic transducers shifted relative to one another is used, such that a maximum transverse irradiation of the nth transducer coincides with a minimum transverse irradiation of the (n+1)th transducer, (n=1,2,3,...). A tape-transport mechanism is used with this purpose. The impregnation container is closed to prevent any vapour release to the environment. The impregnation container can be made of stainless steel. The width of the impregnation container is 500 - 3000 mm. In the impregnation container a temperature control sensor is installed.

Fig. 2 illustrates an embodiment of the System of reagent preparation unit-impregnation unit:

1 - impregnation container

2 - pump (for supplying the reagent to the impregnation unit through the temperature control device 6)

3 - reagent filling tap

4 - reagent discharge tap

5 - reagent preparation unit (taps are not shown)

6 - temperature control device.

After impregnation the textile passes between two rollers for extraction and afterwards can be supplied to the secondary treatment unit 4 according to Fig. 1 (if necessary). The secondary treatment may include: vapour or heated

air treatment, additional heating, washing in an alkaline solution or in a different pH solution. The secondary treatment is required for the completion of the sol and the gel formation (i.e formation of nanoparticles) in the event this does not occur in the impregnation container when the temperature of the solution exceeds the threshold value.

The last stage of the treatment is washing with water and drying. Fig. 3 illustrates an example of the washing and drying structure:

1 - container;

2 - frame;

3 - roller;

4 - guide roller;

5 - thermometer;

6 - extraction mechanism;

7 - possible water heating devices;

8 - heaters;

9 - roller;

10 - rotation motor,

11 - basket for the fabric.

The textile goes through the washing container where it passes through water, in this case barbotage may be provided. The washing container is an open container having a width up to 3000 mm. After washing the textile passes between two rollers for extraction. Afterwards the textile is transported over the metal surface with a temperature up to 200 Celsius degrees. The tape-transport mechanism is used for transporting the fabric.

The device as claimed allows for providing the application of antibacterial compositions onto the fibers in a continuous mode. The output of the device can be no lower than 1.5 m/min.

Thus, the claimed invention allows for production of a new more efficient antibacterial material based on the available metal oxides and compositions thereof. A textile treatment technique is suggested as well.

**Claims**

**1.** A three-dimensional antibacterial material being a microfiber matrix with nanoscale compositions chemically or physically attached to its fibers wherein nanoscale compositions are represented by nanoparticles of titanium oxide (TiIO2) or compositions containing nanoparticles of titanium oxide and nanoparticles of other metal oxides of other metals or their compounds thereof, wherein the content of nanoparticles in the material structure complies with the following inequality:

$$1{,}1 \leq \frac{\sum X_i}{\sum Y_i} \leq 100$$

, where

- Xi - a total concentration of nanoparticles of titanium oxide TiO2
- Yj - a total concentration of nanoparticles of oxides of other metals or compounds thereof,

with a content of nanoscale particles therein from 0.1 % to 30 % (wt), and the size of nanoparticles in the material from 1 nm to 1000 nm.

2. The three-dimensional antibacterial material of claim 1, **characterized in that** it contains ZnO, CuO, MgO, CeO2 as nanoparticles of oxides of other metals or compounds thereof.

3. A method for producing a three-dimensional antibacterial material, including impregnation of a textile material with a titanium-containing reagent, **characterized in that** a solution of the titanium-containing reagent is prepared beforehand, followed by textile material impregnation with a solution prepared under ultrasonic vibrations at a frequency from 10 kHz to 100 kHz until the fibers are completely moistened and impregnated, followed by sol formation from the initial solution with a subsequent formation of gel, and after washing the material mainly with water a thermal treatment is performed at a temperature from 80 to 200 Celsius degrees.

4. The method of claim 3, **characterized in that** the sol formation is performed from the initial solution by heating or preliminary drying.

5. The method of claim 3, **characterized in that** the sol formation is performed from the initial solution by water vapor treatment.

6. The method of claim 3, **characterized in that** the sol formation is performed from the initial solution by adding a pH adjusting/modifying substance.

7. The method of claim 3, **characterized in that** the sol formation is performed from the initial solution through reaching a threshold temperature around the fibers during the material impregnation under ultrasonic vibrations ensuring heating of the sol formation area.

8. A method for producing a three-dimensional antibacterial textile material, including impregnation of the textile material with a titanium-containing reagent, **characterized in that** the textile material is preimpregnated with a reagent having a pH different from the neutral one, the textile material being subsequently treated with the titanium-containing reagent under ultrasonic vibrations at a frequency from 10 kHz to 100 kHz until the fibers are completely moistened and impregnated, wherein the nanosol and gel are formed on the fibers, the material is washed, mainly with water, and dried at a temperature from 80 to 200 Celsius degrees.

9. The method of any of claims 3-8, **characterized in that** the titanium-containing reagent in addition to titanium-containing compounds comprises compounds of other metals (Zn, Cu, Mg, Ce etc.) serving as starting compounds for formation of nanoparticles of oxides of these metals.

10. A device for production of a three-dimensional antibacterial material comprising a reagent preparation unit, an impregnation unit,
wherein the reagent preparation unit comprises a container equipped with taps for adding liquid components, as well as dispensers for adding powder components, a mixing unit, as well as temperature measuring sensors, a temperature control unit for controlling and maintaining temperature below the temperature at which nanoparticles start to form,
wherein the impregnation unit comprises an impregnation container, a reagent heating unit, a temperature control sensor, ultrasonic transducers of a cylindrical or a flat shape, ensuring a uniform exposure of the transported textile on both sides to mechanical vibrations at a frequency 10-100 kHz provided by ultrasonic generators, a tape-transport mechanism for transporting the textile material, the impregnation unit comprising an even number of ultrasonic transducers shifted relative to one another such that the transverse exposure maximum of the nth transducer coincides with the transverse exposure minimum of the (n+1)th transducer, where n = 1,2,3.

11. The device of claim 10, **characterized in that** it further comprises a washing unit and a drying unit.

12. The device of claim 11, **characterized in that** the textile washing unit comprises a mechanism providing barbotage of the liquid.

13. The device of any of claims 10, 11 or 12, **characterized in that** the reagent preparation unit is equipped with a container for storage of initial liquid reagents.

14. The device of any of claims 10 - 13, **characterized in that** it further comprises a secondary treatment unit comprising a vapor treatment module or a liquid reagent impregnation module or a special medium washing module comprising a container and a tape-transport mechanism or a module for preliminary drying at a lower temperature.

**15.** The device of any of claims 10 - 14, **characterized in that** it further comprises a preimpregnation unit placed upstream the impregnation unit, the preliminary impregnation unit comprising a container for impregnation and a tape-transport mechanism for transporting the textile material.

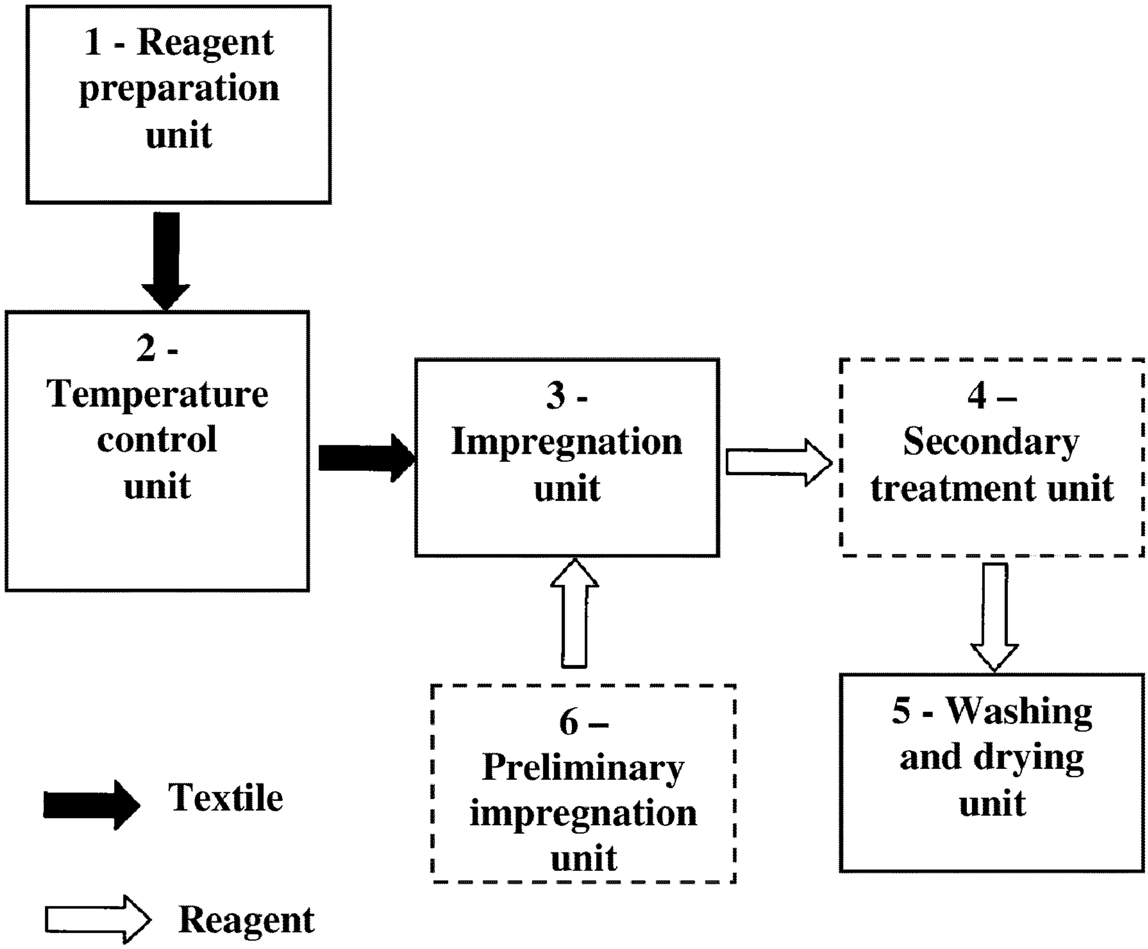
1 - Reagent preparation unit
2 - Temperature control unit
3 - Impregnation unit
4 – Secondary treatment unit
5 - Washing and drying unit
6 – Preliminary impregnation unit
Textile
Reagent

Fig. 1

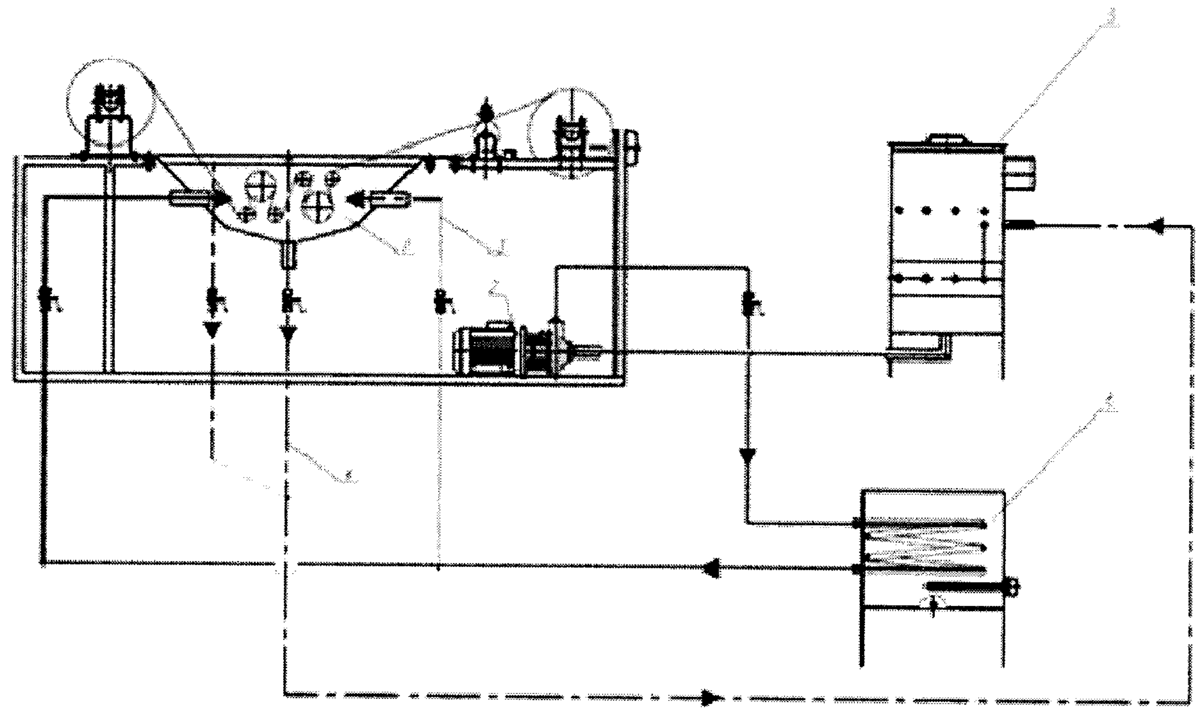

Fig. 2

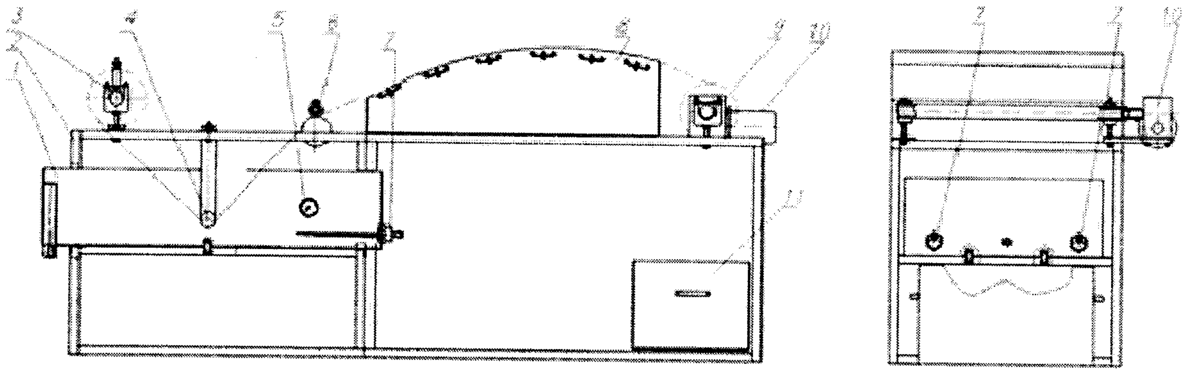

Fig. 3

## INTERNATIONAL SEARCH REPORT

International application No.
PCT/RU 2016/000567

A. CLASSIFICATION OF SUBJECT MATTER
A61K 9/70 (2006.01); A61K 33/08 (2006.01); A61K 33/24 (2006.01); A61P 31/04 (2006.01); B82Y 5/00 (2011.01)
According to International Patent Classification (IPC) or to both national classification and IPC

B. FIELDS SEARCHED

Minimum documentation searched (classification system followed by classification symbols)
A61K 9/00, 9/70, 33/00, 33/06, 33/08, 33/24, A61P 31/00, 31/04, B82Y 5/00, D06M 11/00, 23/00, D06B, D06L, D06P, D21B

Documentation searched other than minimum documentation to the extent that such documents are included in the fields searched

Electronic data base consulted during the international search (name of data base and, where practicable, search terms used)
PatSearch, DWPI, RUPAT, ESP@CENET, PubMed, ScienceDirect

C. DOCUMENTS CONSIDERED TO BE RELEVANT

| Category* | Citation of document, with indication, where appropriate, of the relevant passages | Relevant to claim No. |
|---|---|---|
| X<br>A | CN 101333788 A (UNIV SHANGHAI JIAOTONG) 31.12.2008, the abstract, points 1,3,5 of the claims | 1-2<br>10-13 |
| Y | CN 1807750 A (UNIV ZHEJIANG SCIENCE & TECH) 26.07.2006, points 1-3 of the claims, examples 1-3 | 3-9 |
| Y | CN 102182056 A (UNIV XIAN POLYTECHNIC) 14.09.2011, the abstract, [0001]-[0002], points 1-3 of the claims | 3-9 |
| Y | US 3991236 A (VEPA AG) 09.11.1976, the abstract, the claims | 5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 May 2017 (16.05.2017) | 08 June 2017 (08.06.2017) |
| Name and mailing address of the ISA/<br>RU<br><br>Facsimile No. | Authorized officer<br><br>Telephone No. |

Form PCT/ISA/210 (second sheet) (April 2005)

INTERNATIONAL SEARCH REPORT

International application No.
PCT/RU 2016/000567

C (Continuation). DOCUMENTS CONSIDERED TO BE RELEVANT

| Category* | Citation of document, with indication, where appropriate, of the relevant passages | Relevant to claim No. |
|---|---|---|
| A | RU 2412292 C2 (KHOLMATOV TAKHIR KHUSANOVICH) 20.07.2010, the abstract, page 5, lines 26-50, page 6, lines 44-52, figure | 10-13 |
| A | Dytnersky JU.I. Protsessy i apparaty khimicheskoi tekhnology: Uchebnik dlya vuzov. V 2-kh kn.: Chast 1. Teoreticheskie osnovy protsessov khimicheskoi tekhnologii. Gidromekhanicheskie i teplovye protsessy i apparaty. M.: Khimiya, 1995 - 400 s, ss. 155-156 | 10-13 |
| A | RU 2517121 C2 (GOSUDARSTVENNOE NAUCHNOE UCHREZHDENIE INSTITUT EKSPERIMENTALNOI VETERINARY SIBIRI I DALNEGO VOSTOKA ROSSYSKOI AKADEMY SELSKOKHOZYAISTVENNYKH NAUK et al.) 27.05.2014 | 1-13 |

Form PCT/ISA/210 (continuation of second sheet) (April 2005)

**INTERNATIONAL SEARCH REPORT**

International application No.
PCT/RU 2016/000567

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.: 14-15
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 10158433 B4
- DE 102006053326 A1
- WO 2157868 A
- WO 200610049069 A


### Non-patent literature cited in the description


- *Asian Pacific Journal of Tropical Biomedicine,* July 2015, vol. 5 (7), 509-514
- *Materials Letters,* 2013, vol. 96, 121-124